# EUROPEAN PATENT SPECIFICATION

(11) **EP 1 377 837 B1**
(45) Date of publication and mention of the grant of the patent: **16.11.2005**
(21) Application number: 01273985.0
(22) Date of filing: 30.11.2001
(51) Int. Cl.: G01N 35/00, B01L 3/02, B01J 19/00

(54) **ARRAY-BASED BIOMOLECULE ANALYSIS**
BIOMOLEKÜLANALYSE AUF ARRAY-BASIS
ANALYSE DE BIOMOLECULES PAR JEUX

(30) Priority: 16.03.2001 AU PR378001
(43) Date of publication of application: 07.01.2004
(73) Proprietor: Proteome Systems Ltd., North Ryde, NSW 2113 (AU)
(72) Inventor: SLOANE, Andrew, Balmain, NSW 2041 (AU); DUFF, Janice, Kingsford, NSW 2032 (AU); PLUSKAL, Malcolm, Acton, MA 01720 (US); GOOLEY, Andrew, Turramurra, NSW 2074 (AU)
(74) Representative: Smith, Norman Ian
(86) International application number: PCT/AU2001/001562
(87) International publication number: WO 2002/075321

(56) References cited:
- WO-A-00/79238
- WO-A-97/44134
- WO-A-98/23950
- WO-A-98/47006

## Description

### Field of the Invention

This invention relates to the analysis of samples of biomolecules, particularly proteins.

### Background of the Invention

There has been much discussion in recent literature on the development of a "protein chip". Broadly, these are protein arrays (commonly called micro arrays). The current vision is for a protein micro array that will be able to measure thousands of proteins simultaneously, protein - protein interactions, small molecule interactions and enzyme substrate reactions.

Most current approaches to developing a protein chip rely on immobilising proteins on a substrate typically using surface chemistry to immobilise the proteins. The substrate for the chip may be a silicon wafer but other material such as aluminium wafers and glass have been used or proposed for use as a substrate. After the substrate has been prepared it is necessary to attach a protein capture agent such as an antibody, to the chip. In one approach proposed by one Californian company, Zyomyx Inc., the substrate is coated with a thin organic film, an attachment tag is added to the top organic layer and a protein capture agent such as an antibody fragment or peptide is bound to the free end of the tag.

The current strategy is to have knowledge of what is being layered down on the chip surface, such as antibodies, or in some cases, known expressed proteins that are individually purified by affinity capture and then immobilised.

Other methods have been proposed for laying down antibodies. However the task of laying down antibodies or other protein capture agents is far from straightforward. A further problem arises in that proteins are much less robust than DNA and are fragile and will denature if they are treated harshly. Proteins are also extremely sensitive to the physical and chemical properties of the particular substrate.

There are major drawbacks with existing "protein chips". First, proteins have to be bonded to the chip in position in the array. As discussed above, this is typically done by using either arrayed antibodies, such as monoclonal antibodies, which are slow and expensive to produce, or using arrayed antigens. However, the speciticity of the bound antigens and or the bound antibodies in particular, is not high.

A second problem is that the use of a single antibody cannot address the issue of a protein having a number of isoforms. It is possible that not all isoforms will be biologically active. There is a possibility that biologically active isoforms may be swamped by non-active isoforms.

There is a major problem with abundant proteins in a sample causing high background "noise" by non-specific binding to the array.

One solution, the use of recombinant antigens, does not produce authentic modifications as the recombinant protein will almost certainly have different post translational modifications to the authentic protein. Thus, it impossible to be sure that the interaction on the protein chip is anything like a "real" interaction as would take place between an authentic protein and, for example, another protein

The present invention seeks to address and alleviate the problems of the prior art as discussed above.

### Summary of the Invention

According to the present invention there is provided a method for simultaneously carrying out a plurality of analyses as defined in claim 1.

In a first broad aspect the present invention involves the step of generating an array of macromolecules and subsequently transferring the array of macromolecules to a support. This step generates a primary or macro array. One major advantage of this process is that authentic macromolecules can be arranged and immobilised without any chemistry for the immobilisation process as is required in the prior art "protein chips".

The next step of the process of the present invention "prints" a secondary (or micro) array of reagents onto each coordinate of the captured primary array typically by using image capture to define the coordinates. Apparatus which can be used to perform this function is described in Australian patent No 7225 78 entitled "Analysis of Molecules" which describes an apparatus for capturing an image of an array of spots on a planar support and using that image to drive a print head to a particular spot to apply a reagent to that spot

The expression macromolecules covers any biomolecules selected from the group consisting of proteins, peptides, saccharides, lipids, nucleic acid molecules, complex biomolecules including glycoproteins, and mixtures thereof.

The biomolecules are preferably separated by chromatography to form an array of samples. The chromatography is preferably electrophoresis, and more preferably electrophoresis carried out in a polyacrylamide gel.

The electrophoresis can be carried out in one dimension including isoelectric focusing, native polyacrylamide gel electrophoresis, and sodium dodecyl sulfate (SDS) polyacrylamide gel electrophoresis. Alternatively, the polyacrylamide gel electrophoresis is carried out in two dimensions with the first dimension by isoelectric focusing and the second dimension is by native polyacrylamide gel electrophoresis or SDS polyacrylamide gel electrophoresis.

It is preferred to utilise a non-denaturing electrophoresis separation process whenever possible as this generates a more authentic array of native, rather than de-natured proteins.

The support may be a membrane made of polyvinylidene difluoride, nitrocellulose, nylon, teflon, zitex, polypropylene, PTFE, and derivatised forms thereof having one or more functional groups.

Image analysis can determine the maximum number of spots that can be practically printed in the micro array as well as determining the individual spot-spot resolution for each molecule in the macro array.

The process of identifying an interaction that is specific or non-specific would follow methods in the public domain.

However, one could use a unique feature of the printing process where non-specific interactions are washed to an outer corona whereas specific interactions remain focused on the coordinate deposited. The existence or otherwise of specific interaction, is thus disclosed by the absence or presence of a corona.

Detection of the protein can be assisted by direct labelling with a marker or the like or use of a sandwich technique such as second Antibody labelled fluorescence.

The next step in the process is the use of detection means to detect whether interactions have taken place between the protein spot and the reagent or reagents applied by the chemical printer. Detection may be carried out by any suitable means such as a global capture lens such as a CCD, camera, scanning or laser scanning, microscopy or the like.

In an alternative embodiment a detection means may be driven directly to each coordinate of the micro array.

It is envisaged that the process of the present invention could be used for batch mode purification of expressed proteins containing an affinity tag. For example, a batch of say 384 clones expressing a specific but different Histag protein may be purified over an IMAC (immobilised metal affinity chromatography) column. The eluate from the column (i.e. all 384 clones) may then be arrayed using 2D electrophoresis and transferred to a substrate so as to generate a non-predetermined array. This is in direct contradiction to the existing teaching in the art, which relies on maintaining a pre-determined array and retaining positional information. One advantage of this example is that the arraying of the expressed proteins would provide a means of quality control of the expressed product compared to the predicted product (for example the predicted Mr and pI compared to the observed Mr and pI).

The principal advantage of the present invention over the prior art is that is that it generates an array of authentic proteins without the need for surface immobilisation chemistry as is required for existing protein chips.

In one feature, the information contained in the image of the primary array can be used to define the type of micro array printed on the primary array. For example, the size of a particular spot to be analysed can be used to determine the pattern, and spacing of reagents dispensed onto that spot For example, an 8 x 8 array of reagents with 20 micron drops can be printed on a spot having a 200 microns diameter with the reagent spots spaced 25 microns apart whereas with a larger say 400 microns spot the reagent spots may be spaced 50 microns apart.

Since there is sufficient accuracy in the depositing system it is possible to print very high density arrays onto individual positions of the primary array and with precision fibre optics it should be possible to identify minute interactions.

In one particularly preferred feature, it would be an advantage to print multiple proteolytic enzymes as the micro array onto particular protein spots of the macro array. For example, on a 500 micron diameter protein spot a micro array of a number of endoproteinase enzymes (trypsin, endoproteinase LysC, endoproteinase GluC and endoproteinase Asp-N, with the preferred enzymes being trypsin and GluC,) is printed at 200 micron size spots spaced 200 microns apart (centre to centre). The spot size and spacing is sufficiently small enough so that the average MALDI-TOF-MS nitrogen laser beam (100 micron) can be positioned so as to only desorb the analytes of one particular enzyme reaction within a spot of the macro array. The advantage of this feature is that the micro array of proteinases would lead to an increased peptide coverage detected during MALDI-TOF-MS analysis of the protein spot in the macro array.

### Brief Description of the Drawings

Specific embodiments of the present invention will now be described, by way of example only, and with reference to the accompanying drawings in which:
Figure 1 illustrates an array of proteins which have been separated by 2D electrophoresis and transferred onto a solid support membrane;
Figure 2 illustrates a chemical printer being used to deposit a series of micro arrays of small spots on top of a protein spot identified in the array shown in Figure 1;
Figure 3 is a close up of the spot shown in Figure 1 showing a micro array deposited onto that spot;
Figure 4 is a schematic representation showing the process of obtaining information on an array of components or samples by way of acquiring or recording an image of the position of at least one component or sample in the array and utilising the recorded image so as to allow the manipulation of the at least one component or sample *in situ.*
Figure 5 is a schematic representation of equipment for imaging, manipulating and analysing at least one component or sample of an array of components or samples.
Figure 6a to 6c illustrate apparatus for clamping a nitrocellulose (NC) membrane;
Figures 7a and 7b illustrate the effect of micro-jetting TB negative or positive human serum onto a 38 kDa TB antigen;
Figure 8 illustrates the effect of micro-jetting TB negative or positive human serum onto a denatured 38 kDa TB antigen with and without Direct Blue Staining;
Figure 9 shows a Membrane blot B745 adhered with double-sided tape to Axima MALDI target plate; and
Figure 10 is a close up of protein digested with trypsin and GluC endoproteases with matrix deposited on top.

### Detailed Description of a Preferred Embodiment

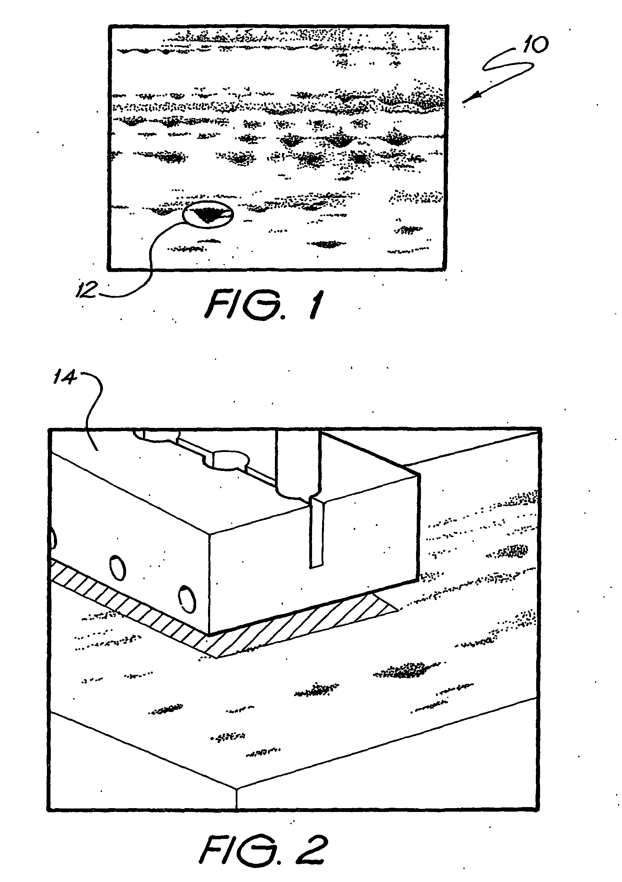

In the present invention, a mixture of proteins is fractionated to remove abundant proteins and a narrow range of pH gradient is used to resolve isoforms (1D electrophoresis). The fractionation process may be carried out using a multi compartment electrolyser such as is described in International Patent Application No PCT/AU00/01391. Instead of 1D electrophoresis the sample after removal of abundant proteins may be separated out into an array (10) illustrated in Figure 1 using 2D electrophoresis or the like and the array is then transferred using electrophoretic blotting onto a membrane such as an nitrocellulose membrane. The array of proteins is now immobilised and ready to be treated as a "chip". It is to be noted that the array is not predetermined and it is not necessary at the time the array is generated to know which proteins are located in which position on the array.

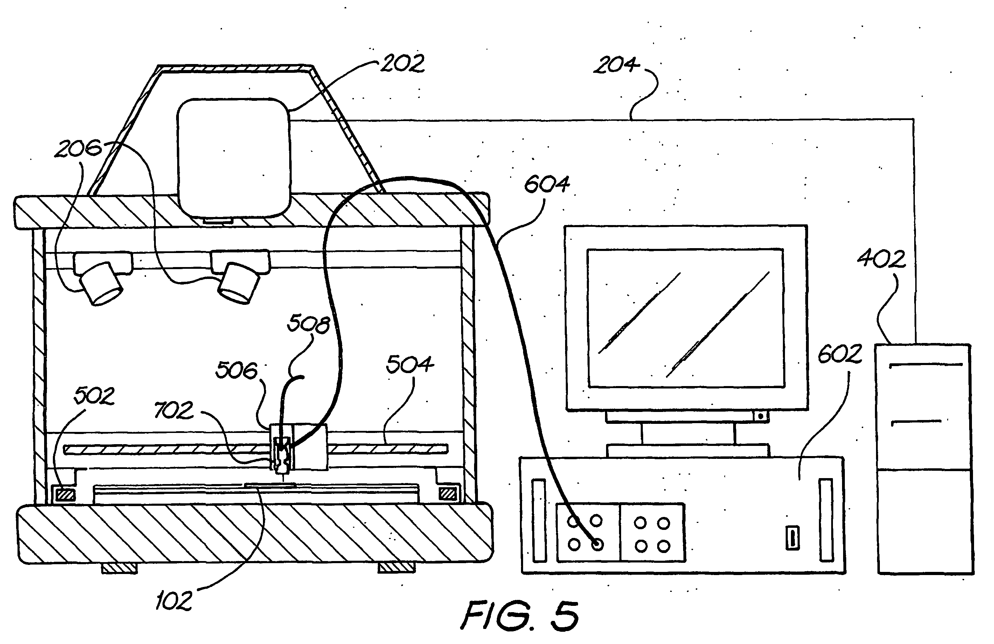

Figure 1 illustrates a protein spot 12 which is ringed. The next step in the process is printing an array of reagents onto the protein spot This is done by using the chemical printer described in AU 722578 (WO 98/47006) which is described below with reference to Figures 4 and 5.

Figure 4 shows a schematic representation of the printer system function. The system comprises an array 100, an image acquisition system 200, an image analysis system 300, a computer 400, an x,y,z adjustable platform 500, a plurality of chemical dispensing control units 600, a plurality of dispensing heads and reservoirs 700, an analyser control unit 800, an analyser 900, and a data analysis station 910.

The array 100 is positioned on or under the x,y,z adjustable table or arm 500 and an image 200 is acquired and transferred to the computer 400 as a digital image. This image is either interpreted by an image analysis system 300 where the coordinates of each component of the array are transformed to values that reflect the true x, y, z axes. Alternatively, the image stored in the computer 400 is used without interpretation and the coordinates of one particular component within the array 100 are used to move the x,y,z adjustable table or arm 500 which carries a dispensing head (jetting device) 700. The dispensing head 700 is under the control of a chemical dispensing control unit 600 which is controlled by the computer 400 and dispenses a reagent onto the selected sample in the array 100. When the treatment has been completed, the coordinates of the treated component within the array 100 are used to move the x,y,z adjustable table or arm 500 which carries a analyser 900. The analyser 900 is under the control of an analysis control unit 800 which when selected by the operator via the computer 400 analyses treated selected sample 100. Data from the analysis is then collated by a data analysis and management system 910 which is correlated with the interpreted coordinates of each sample in the array from the image analysis system 300.

The x,y,z, adjustable platform, a chemical dispensing control unit, a dispensing head and reservoir, and an analyser, all under the control of a computer, are shown in Figure 5. The array 102 is fixed onto a platform 502. The image of the array 102 is acquired via a digital camera 202. The array 102 is illuminated via a camera flash or external tungsten lamps 206. The image is transferred from the camera 202 to the computer 402. The image is processed and imported into "click-on-a-spot" software. This process translates the image pixel coordinates into robot coordinates. The "click-on-a-spot" software is then used to drive the dispensing device 702 to the selected sample in the array via an x,y movable bar 504. The z movement of the dispensing device 702 is via the dispensing device support unit 506. Reagent is dispensed from the reagent reservoir 508 via the computer control 402 of the chemical dispensing control unit 602 which is directly linked 604 to the dispensing device 702.

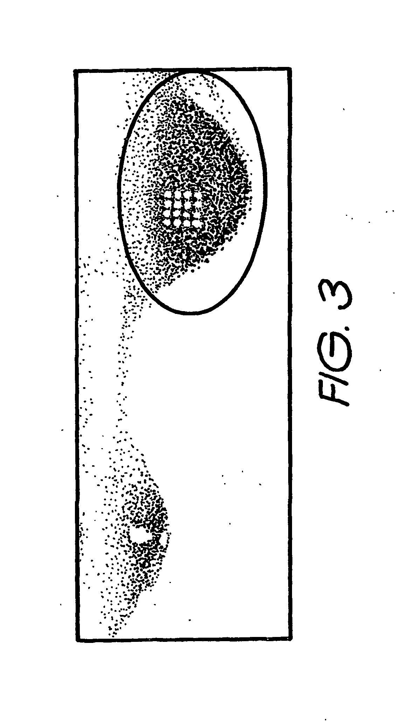

Figure 2 illustrates the dispensing device 702 in the form of a printer head 14 moving above the protein spot 12 to deposit spots of reagent onto the spot 12. The printer utilises piezo electric printing to leave a very small quantities of liquid (of the order of pl) on top of the spot without contacting the spot.

The print head 14 is directed to the spots using a previously generated image of the array which provides the xy coordinates for the particular spots in the array, using the technique described in AU 722578 and repeated above.

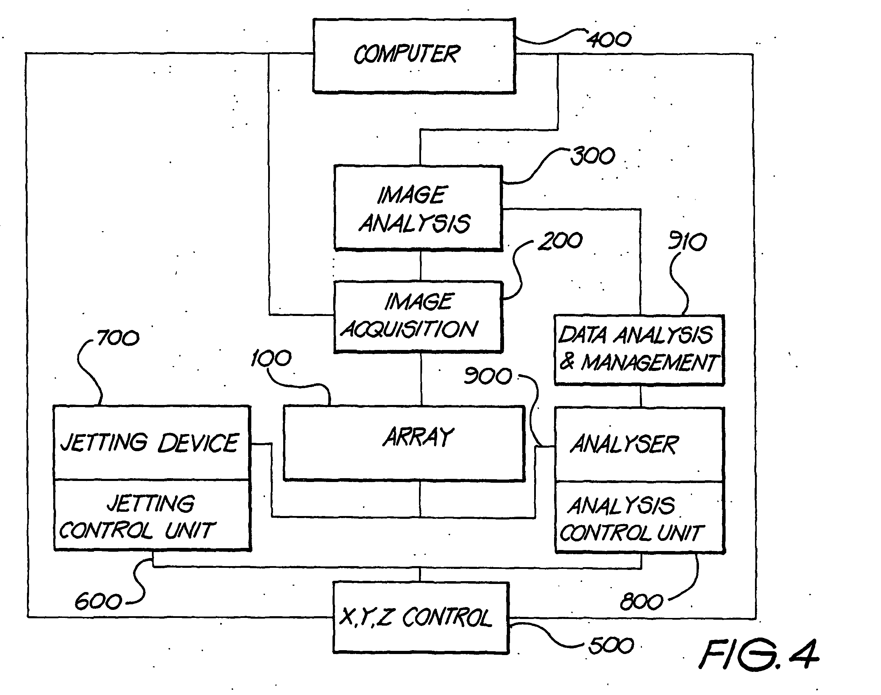

Figure 3 shows an image of a 4 x 4 array of reagents deposited onto the protein spot 12.

Thus, in contrast with the prior art techniques, the protein chips of the present invention provide authentic protein arrays, the ability to resolve the issues of isoforms and the technique for the removal of abundant proteins. One particular technique which is envisaged, is to allow patients to make antibodies and printing patient sera onto protein arrays.

The potential uses of the present invention include the use of antibodies screening for new antigens, measuring peptide/protein interactions, and protein/protein interactions.

### Example: 1

The aim of this example was to develop chemical printing technology for micro-dispensing human serum that is either seronegative or seropositive for *Mycobacterium tuberculosis* (TB) as an approach for defining patient immunoreactivity for TB using a purified TB antigen on a nitrocellulose matrix. It will be appreciated that this approach could be used for defining patent immunoreactivity to a number of conditions or diseases by using appropriate antigens.

### Materials and Methods 1.1

Human serum isolated from 2 patients, one seronegative and the other seropositive for TB, was diluted 1 in 10 using PBS, pH 7.4 + 0.05% (w/v) sodium azide + 0.1% (v/v) Tween-20 (PBS wash buffer (PBS-WB)) and then filtered through a 0.22 µm syringe filter (Millipore, North Ryde, Australia). 4 ul of a 370 µg/ml solution of purified 38 kDa TB antigen in PBS, pH 7.4 was applied onto a nitrocellulose membrane (Bio-Rad, Hercules, CA) and then allowed to dry. Non-specific binding sites on the nitrocellulose were then blocked for 15 min using 0.5% (w/v) casein (Sigma, Castle Hill, Australia) in PBS-WB. A 4 x 4 array, at 100 drops per spot, of each serum sample was then micro-jetted onto separate TB antigen spots using an AB-55 microjet device (MicroFab Technologies, Plano, Texas), #B0-13-12 with a 55 µm orifice at a frequency of 240 Hz The nitrocellulose was kept moist during the dispensing of serum by underlaying it with filter paper saturated with PBS-WB. Ten seconds after the serum had been jetted, the nitrocellulose was rinsed with several drops of PBS-WB using a transfer pipette. The nitrocellulose was subsequently incubated for 1 hour with anti-human IgG conjugated to FITC (Zymed, San Francisco, CA), at a 1 in 100 dilution in 0.5% (w/v) casein/PBS-WB, pH 7.4 followed by washing with PBS-WB. Labelled antigen was detected using a Bio-Rad FluorS™ Multi-Imager (Hercules, CA).

### Materials and Methods 1.2

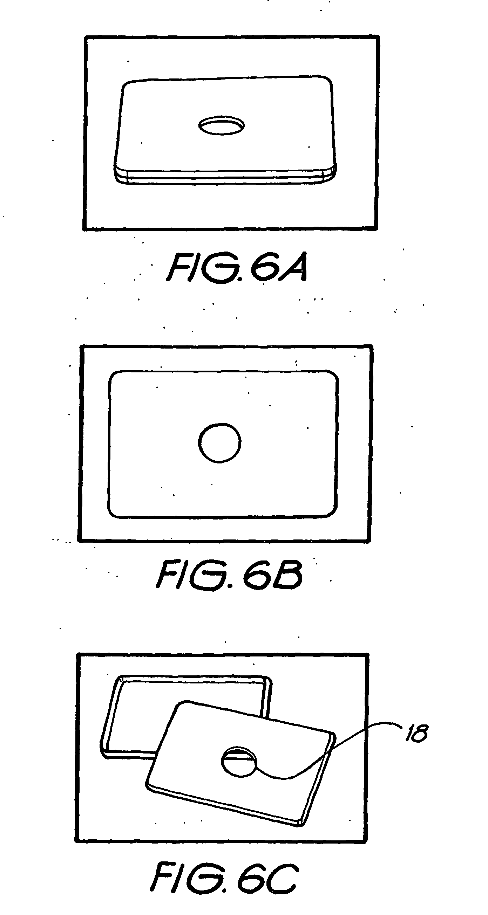

The above method was repeated except that the nitrocellulose was underlayed with absorbent tissue using the apparatus illustrated in Figures 6a to 6d. Absorbent tissue paper was packed beneath a nitrocellulose membrane containing TB antigen. This material was all then clamped shut inside the apparatus shown in Figures 6a to 6c with the area to be jetted on exposed at the circular orifice 18. The tissue paper underlay ensured that jetted solution or any applied small volume of buffer or reagent was immediately pulled through the nitrocellulose into the tissue paper allowing for an instantaneous and specific reaction. This approach prevented both non-specific drying of immunoglobulin, and hence non-specific reactions, and also prevented dispersing of specific antibody across the surface of the nitrocellulose (see below). In contrast to method 1, this device kept the nitrocellulose membrane dry during jetting. The membrane was then treated with 1 drop of PBS-WB using a transfer pipette and serum then jetted onto the antigen as described above.

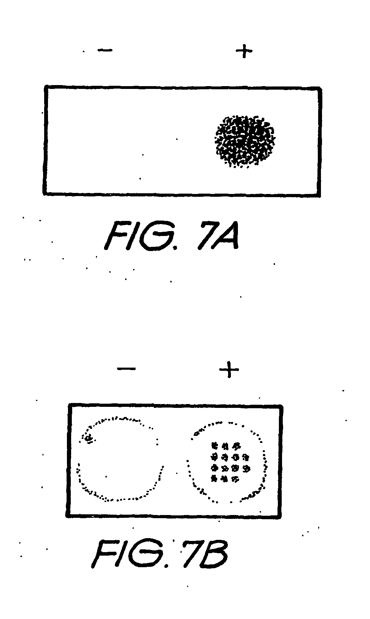

Figures 7a and 7b illustrate the effect of micro-jetting TB negative or positive human serum onto a 38 kDa TB antigen. A 4 x 4 array 20 of human serum either seronegative (-) or seropositive (+) for TB was jetted onto nitrocellulose containing 4 µl spots of 1.48 µg 38 kDa TB antigen. The nitrocellulose membranes were underlayed with either filter paper moistened with PBS-WB (A) or with tightly packed dry absorbent tissue paper (B). Labelled antigen was detected using FTTC-labelled secondary antibody followed by analysis with a BIO-RAD FLUOR-S™ Multi-Imager. It is clearly evident that the tissue paper packing and dry membrane shown in Figure 7b at 20 resulted in increased sensitivity and resolution of antigen detection.

### Example 2

The development of chemical printing technology for micro-dispensing human serum that is either seronegative or seropositive for *Mycobacterium tuberculosis* (TB) as an approach for defining patient immunoreactivity for TB using a purified TB antigen subjected to SDS-PAGE and electrotranferrance to nitrocellulose with/without subsequent Direct Blue staining.

### Materials and Methods 2

14.8 µg of 38 kDa TB antigen was diluted to 200 ul using x1 SDS-PAGE non-reducing sample buffer. Sample was then analysed by SDS-PAGE (1.48 µg of antigen per lane) using a 4-12% (w/v) Tris-Bis polyacrylamide gradient gel (Invitrogen, Carlsbad, CA) followed by electrotransferrance to nitrocellulose. Two lanes of the blot were visualised using Direct Blue stain (Fig. 3) whilst the other two lanes were not stained. Both blots were allowed to dry at room temperature and were subsequently blocked with 0.5% (w/v) casein in PBS-WB for 15 min. Both blots were then rinsed with PBS-WB and allowed to dry. Blots were then mounted into the suction device described in Fig. 1 and seronegative or seropositive TB serum then jetted onto alternate 38 kDa bands of the Direct Blue stained blot and the non-stained blot as a 1 x 5 array as described above. Approximately 10 seconds later blots were washed with 2 drops of PBS-WB using a transfer pipette. 5 µl of FITC-labelled secondary antibody diluted 1 in 10 with 0.5% (w/v) casein/PBS-WB was then applied to the blot followed 10 seconds later by two drops of PBS-WB using a transfer pipette. Labelled antigen was detected using a BIORAD FluorS™ Multi-Imager (Hercules, CA).

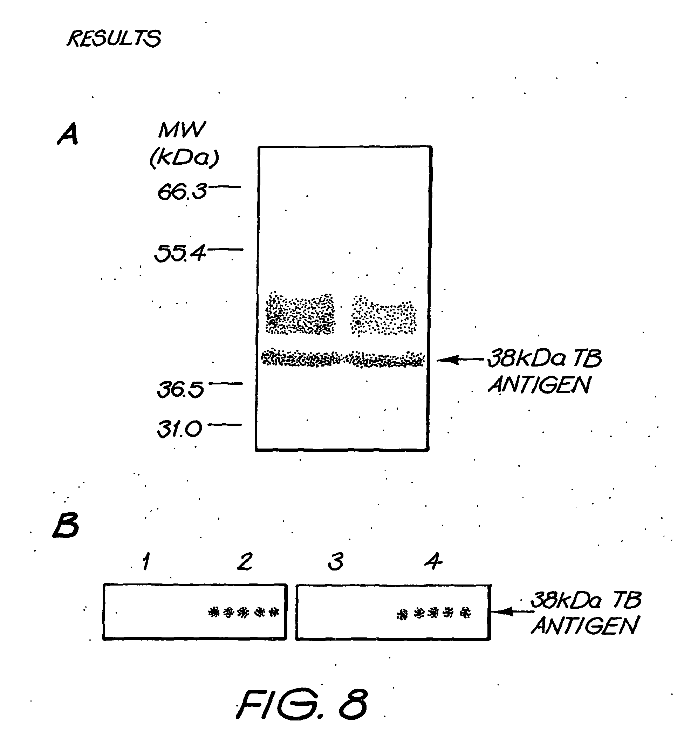

Figure 8 illustrates the effect of micro-jetting TB negative or positive human serum onto a denatured 38 kDa TB antigen +/- with or without Direct Blue Staining. (A) 38 kDa TB antigen, 1.48 µg per lane, was separated by SDS-PAGE using a 4-12% polyacrylamide gradient gel. Protein was then electrotransferred onto nitrocellulose. Two of these lanes were then stained with Direct Blue. (B) After blocking with 0.5% (w/v) casein/PBS-WB, a 1 x 5 array of human serum either seronegative (lanes 1 and 3) or seropositive (lanes 2 and 4) for TB was jetted (as described above) onto the 38 kDa TB antigen band. The nitrocellulose membranes were underlayed with tightly packed dry absorbent tissue paper during jetting using the apparatus shown in Figures 6a to 6c. Only Lanes 3 and 4 had been stained with Direct Blue prior to jetting serum (A). Labelled antigen was detected using FITC-labelled secondary antibody followed by analysis with a BIO-RAD FLUOR-S™ Multi-Imager.

The current protocols using a chemical printer to dispense nanolitre volumes of human serum to a defined antigen immobilised on a nitrocellulose membrane have proven very successful and provide an extremely rapid means of detecting TB-immunoreactive IgG (less than 1 minute). No background or non-specific binding was observed. Initial studies demonstrated that dispersal of serum antibody across the surface of a moist nitrocellulose membrane after jetting created poor resolution of antibody arrays. The implementation of absorbent tissue paper beneath a dry nitrocellulose membrane has effectively overcome this problem, and now permits highly specific and well-resolved antibody arrays:

### Example 3

This example concerns the application of enzymes to proteins on an array prior to matrix assisted laser desorption ionisation (MALDI) analysis of the fragmented protein in a mass spectrometer. As is known different enzymes cleave proteins at different amino acid sites. Some enzymes such as lysC, AspN, ArgC cleave at only one specific amino acid site. This is a problem in MALDI analysis as it produces a few large fragments which tends not to produce a very informative spectrum. Other enzymes such as pepsin and Chymotrypsin cleave proteins cleave at many amino acid sites. Use of these enzymes prior to MALDI analysis is also problematic as too many small fragments are produced which produce a large number of very small peaks in the spectrum and which are again very difficult to interpret. Trypsin and GluC cleave at two amino acid sites and this tends to produce good spectra for analysis and thus are the enzymes of choice in MALDI analysis.

Even so, because these enzymes only cleave at specific amino acid sites use of a single enzyme produces a limited amount of information or coverage on the protein. However jetting two different enzymes onto a protein spot in an array using the method of the present invention will increase the coverage of the protein and the experiment below describes the use of the method of the present invention to jet both Trypsin and GluC onto two human proapohpoprotein to obtain improved coverage (matched peptides) using both GluC and Trypsin compared with Trypsin alone or GluC alone.

The aim of this example was to develop chemical printing technology for micro dispensing multiple endoproteases onto purified proteins subjected to SDS-PAGE and electroblotted onto polyvinyl difluoride membranes with Direct Blue staining to improve protein identification.

### Materials and Methods

The sample was 36µl of whole plasma in 7M urea,2M thiourea, 2% (w/v) Chaps and 5mM Tris. The sample was reduced with X tributylphosphine and alkylated with z iodoacetamide. The sample was ultra sonicated and then centrifuged where the supernatant was collected. Prefractionation was performed with the multicompartment electrolyzer (MCE) using methods described in Herbert, B. & Righetti, P. G. A turning point in proteome analysis: sample prefractionation via multicompartment electrolyzers with isoelectric membranes. *Electrophoresis* **21,** 3639-3648 (2000), the entire contents of which are incorporated herein by reference.

Dry 11cm 5-6 IPG strips were rehydrated for 6hrs with 200µl of protein sample. Rehydrated strips were focused for 120kVhr at a maximum of 10000V. The focused IPG strips were equilibrated for 20 mins in equilibration buffer containing 6 M urea, 2% (w/v) SDS.

The Equilabrated IPG strips were inserted into the loading wells of 6-15% gel chips. Electrophoresis was performed at 50mA per gel for 1.5hrs. Proteins were electroblotted onto an Immobilon P^{SQ} PVDF membrane at 400mA for 1hr and 20 mins. Proteins were stained with Direct Blue 71.

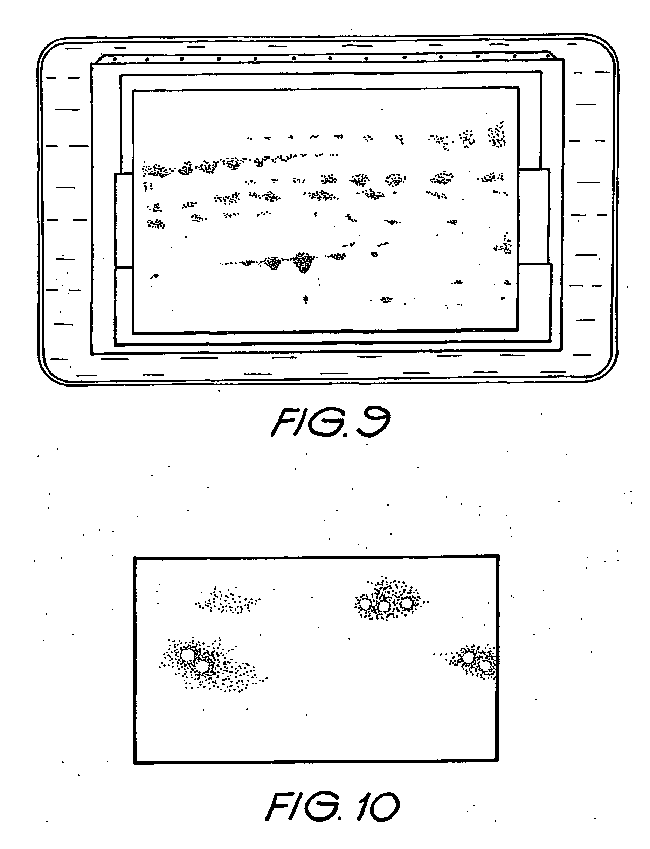

The Membrane blot was then adhered to an Axima-CRF MALDI-TOF MS target plate using 3M double-sided conductive tape (refer to Fig 9). The specified protein spots were blocked with 5ηl 1% polyvinylpyrrolidonein 50% methanol, dispensed at 50 drops, 300µm in diameter, onto two positions, 1mm apart, on the one protein spot. Excess PVP was rinsed of with MilliQ. 50ηl of 200µg/ml GluC endoprotease was jetted onto one of the PVP spots on the protein and X amount of 200µg/ml of trypsin was jetted onto the remainder PVP spot on the same protein. The membrane plate was then placed in a sealed lunchbox with minimal water to create a humidified environment and incubated at 37°C for 3hrs. After digestion, 100ηl of 10 mg/ml of α-cyano-hydroxycinnamic acid in 20% isopropanol, 20% 2-butanol, 30% methanol and 0.5% formic acid was jetted onto the digested protein spots (Fig 10). The digest was analyzed using the Axima CRF MALDI-TOF mass spectrometer.

The current procedure to microdispense multiple endoproteases to increase amino acid coverage for protein identification had proven successful by an combined coverage of 66.67% of matched peptides compared to 40% gluC and 46.09% trypsin coverage achievable independently.

The matrix solution used comprises 20% 2-Butanol, 20% iso-propanol, 30% methanol, 30% aqua and 0.1%Tfa(trifluoroacetic acid). This matrix solution has the advantage that it has a suitable viscosity to dispense stable drops over an extended period of time.

It will be appreciated by persons skilled in the art that numerous variations and/or modifications may be made to the invention as shown in the specific embodiments. The present embodiments are, therefore, to be considered in all respects as illustrative and not restrictive.

## Claims

1. A method for simultaneously carrying out a plurality of analyses on a plurality of macromolecules, wherein the macromolecules are in a primary array of spots on a support, the method comprising:
1. applying a plurality of compositions comprising one or more reagents, each composition applied to form a spot of a secondary, smaller array of spots; the secondary, smaller array of spots being within each spot of the primary array of macromolecules; and
2. detecting whether interactions have taken place between the macromolecules of the primary array and the reagent or reagents in the composition applied in the secondary array.

2. The method of claim 1 wherein the primary array is non-predetermined.

3. The method of any preceding claim wherein the primary array is generated on a planar support without surface immobilisation chemistry.

4. The method of any preceding claim wherein the primary array is generated by chromatography.

5. The method of claim 4 wherein the chromatography is electrophoresis.

6. The method of claim 5 wherein the electrophoresis is polyacrylamide gel electrophoresis.

7. The method of any one of claims 4 to 6 wherein the chromatography is in two dimensions, the first dimension employing isoelectric focusing and the second dimension employing non-denaturing polyacrylamide gel electrophoresis or sodium dodecyl sulphate polyacrylamide gel electrophoresis.

8. The method of any preceding claim wherein the primary array is generated by chromatography followed by transfer to a membrane.

9. The method of claim 8 wherein the membrane is polyvinylidene difluoride, nitrocellulose, nylon, Teflon™, Zitex™, polypropylene, polytetrafluoroethylene or a derivatised form of any of the foregoing.

10. The method of any preceding claim wherein the macromolecules are proteins, peptides, saccharides, lipids, nucleic acid molecules, glycoproteins or mixtures of any of the foregoing.

11. The method of claim 1 wherein the macromolecules are proteins.

12. The method of claim 11 wherein the macromolecules are proteins from humans or animals.

13. The method of any one of claims 1 to 10 wherein the macromolecules are antibodies.

14. The method of any one of claims 1 to 10 wherein the macromolecules are antigens from bacteria.

15. The method of any preceding claim wherein the reagents are proteins.

16. The method of claim 15 wherein the proteins are from humans or animals.

17. The method of claim 15 wherein the reagents are antibodies.

18. The method of claim 15 or 16 wherein the reagents are enzymes.

19. The method of claim 18 wherein the enzymes are selected from the group consisting of: Lys-C, Glu-C, trypsin, Asp-N, Arg-C, pepsin and chymotrypsin.

20. The method of claim 19 wherein the enzymes are trypsin and Glu-C.

21. The method of claim 1 wherein step b) is performed using MALDI-TOF mass spectrometry.

22. The method of claims 1-21, the method comprising:
generating said primary array of macromolecules as spots on a support.

23. The method of claim 1, the method comprising:
generating a primary array of macromolecules;
transferring the array of macromolecules to a support.

## Patentansprüche

1. Verfahren zur gleichzeitigen Durchführung mehrerer Analysen an mehreren Makromolekülen, wobei die Makromoleküle in einem primären Array aus Punkten auf einem Träger vorliegen, wobei man in dem Verfahren:
1. mehrere, ein oder mehrere Reagentien umfassende Zusammensetzungen aufträgt, so daß jede aufgetragene Zusammensetzung jeweils einen Punkt eines sekundären, kleineren Arrays aus Punkten bildet, wobei das sekundäre, kleinere Array aus Punkten innerhalb eines jeden Punkts des primären Arrays aus Makromolekülen liegt; und
2. nachweist, ob zwischen den Makromolekülen des primären Arrays und dem Reagens bzw. den Reagentien in der im sekundären Array aufgetragenen Zusammensetzung Wechselwirkungen stattgefunden haben.

2. Verfahren nach Anspruch 1, wobei das primäre Array nicht vorbestimmt ist.

3. Verfahren nach einem vorhergehenden Anspruch, wobei das primäre Array auf einem ebenen Träger ohne Oberflächenimmobilisierungschemie erzeugt wird.

4. Verfahren nach einem vorhergehenden Anspruch, wobei das primäre Array miitels Chromatographie erzeugt wird.

5. Verfahren nach Anspruch 4, wobei es sich bei der Chromatographie um Elektrophorese handelt.

6. Verfahren nach Anspruch 5, wobei es sich bei der Elektrophorese um Polyacrylamidgelelektrophorese handelt.

7. Verfahren nach einem der Ansprüche 4 bis 6, wobei die Chromatographie in zwei Dimensionen vorliegt, wobei bei der ersten Dimension isoelektrische Fokussierung und bei der zweiten Dimension nichtdenaturierende Polyacrylamidgelelektrophorese oder Natriumdodecylsulfat-Polyacrylamidgelelektrophorese eingesetzt wird.

8. Verfahren nach einem vorhergehenden Anspruch, wobei das primäre Array mittels Chromatographie mit anschließender Übertragung auf eine Membran erzeugt wird.

9. Verfahren nach Anspruch 8, wobei es sich bei der Membran um Polyvinylidendifluorid, Nitrocellulose, Nylon, Teflon™, Zitex™, Polypropylen, Polytetrafluorethylen oder eine derivatisierte Form eines der vorstehenden Stoffe handelt.

10. Verfahren nach einem vorhergehenden Anspruch, wobei es sich bei den Makromolekülen um Proteine, Peptide, Saccharide, Lipide, Nukleinsäuremoleküle, Glycoproteine oder Gemische aus einem der vorstehenden Moleküle handelt.

11. Verfahren nach Anspruch 1, wobei es sich bei den Makromolekülen um Proteine handelt.

12. Verfahren nach Anspruch 11, wobei es sich bei den Makromolekülen um Proteine aus dem Menschen oder aus Tieren handelt.

13. Verfahren nach einem der Ansprüche 1 bis 10, wobei es sich bei den Makromolekülen um Antikörper handelt.

14. Verfahren nach einem der Ansprüche 1 bis 10, wobei es sich bei den Makromolekülen um Antigene aus Bakterien handelt.

15. Verfahren nach einem vorhergehenden Anspruch, wobei es sich bei den Reagentien um Proteine handelt.

16. Verfahren nach Anspruch 15, wobei die Proteine aus dem Menschen oder aus Tieren stammen.

17. Verfahren nach Anspruch 15, wobei es sich bei den Reagentien um Antikörper handelt.

18. Verfahren nach Anspruch 15 oder 16, wobei es sich bei den Reagentien um Enzyme handelt.

19. Verfahren nach Anspruch 18, wobei die Enzyme ausgewählt sind aus der Gruppe bestehend aus: Lys-C, Glu-C, Trypsin, Asp-N, Arg-C, Pepsin und Chymotrypsin.

20. Verfahren nach Anspruch 19, wobei es sich bei den Enzymen um Trypsin und Glu-C handelt.

21. Verfahren nach Anspruch 1, wobei Schritt b) unter Verwendung von MALDI-TOF-Massenspektrometrie durchgeführt wird.

22. Verfahren nach den Ansprüchen 1-21, bei dem man das primäre Array aus Makromolekülen als Punkte auf einem Träger erzeugt.

23. Verfahren nach Anspruch 1, bei dem man:
1. ein primäres Array aus Makromolekülen erzeugt;
2. das Array aus Makromolekülen auf einen Träger überträgt.

## Revendications

1. Procédé pour effectuer simultanément plusieurs analyses sur plusieurs macromolécules, dans lequel les macromolécules se trouvent dans une matrice ordonnée primaire de points sur un support, le procédé comprenant les étapes consistant à :
1. appliquer plusieurs compositions, comprenant un ou plusieurs réactifs, chaque composition étant appliquée pour former un point d'une matrice ordonnée secondaire de points, plus petite ; la matrice ordonnée secondaire de points, plus petite, se trouvant au sein de chaque point de la matrice ordonnée primaire de macromolécules ; et
2. détecter si des interactions ont eu lieu entre les macromolécules de la matrice ordonnée primaire et le ou les réactif(s) dans la composition appliquée, dans la matrice ordonnée secondaire.

2. Procédé selon la revendication 1, dans lequel la matrice ordonnée primaire n'est pas prédéterminée.

3. Procédé selon l'une quelconque des revendications précédentes, dans lequel la matrice ordonnée primaire est générée sur un support planaire, sans produit chimique d'immobilisation de surface.

4. Procédé selon l'une quelconque des revendications précédentes, dans lequel la matrice ordonnée primaire est générée par chromatographie.

5. Procédé selon la revendication 4, dans lequel la chromatographie est une électrophorèse.

6. Procédé selon la revendication 5, dans lequel l'électrophorèse est une électrophorèse sur gel de polyacrylamide.

7. Procédé selon l'une quelconque des revendications 4 à 6, dans lequel la chromatographie est à deux dimensions, la première dimension employant une focalisation isoélectrique et la deuxième dimension employant une électrophorèse sur gel de polyacrylamide non dénaturant ou une électrophorèse sur gel de polyacrylamide à sodium dodécyl sulfate.

8. Procédé selon l'une quelconque des revendications précédentes, dans lequel la matrice ordonnée primaire est générée par chromatographie, suivi d'un transfert sur une membrane.

9. Procédé selon la revendication 8, dans lequel la membrane est en : difluorure de polyvinylidène ; nitrocellulose ; nylon ; Teflon™ ; Zitex™ ; polypropylèrie ; polytétrafluoroéthylène ; ou une forme dérivée de n'importe lequel des éléments précédents.

10. Procédé selon l'une quelconque des revendications précédentes, dans lequel les macromolécules sont des : protéines ; peptides ; saccharides ; lipides ; molécules d'acide nucléique ; glycoprotéines ; ou des mélanges de n'importe lesquels des éléments précédents.

11. Procédé selon la revendication 1, dans lequel les macromolécules sont des protéines.

12. Procédé selon la revendication 11, dans lequel les macromolécules sont des protéines provenant d'êtres humains ou d'animaux.

13. Procédé selon l'une quelconque des revendications 1 à 10, dans lequel les macromolécules sont des anticorps.

14. Procédé selon l'une quelconque des revendications 1 à 10, dans lequel les macromolécules sont des antigènes provenant de bactéries.

15. Procédé selon l'une quelconque des revendications précédentes, dans lequel les réactifs sont des protéines.

16. Procédé selon la revendication 15, dans lequel les protéines proviennent d'êtres humains ou d'animaux.

17. Procédé selon la revendication 15, dans lequel les réactifs sont des anticorps.

18. Procédé selon la revendication 15 ou 16, dans lequel les réactifs sont des enzymes.

19. Procédé selon la revendication 18, dans lequel les enzymes sont choisies dans le groupe composé des : Lys-C ; Glu-C ; trypsine ; Asp-N ; Arg-C ; pepsine ; et chymotrypsine.

20. Procédé selon la revendication 19, dans lequel les enzymes sont la trypsine et la Glu-C.

21. Procédé selon la revendication 1, dans lequel l'étape b) est effectuée en utilisant une spectrométrie de masse MALDI-TOF.

22. Procédé selon les revendications 1 à 21, le procédé comprenant l'étape consistant à générer ladite matrice ordonnée primaire de macromolécules comme des points sur un support.

23. Procédé selon la revendication 1, le procédé comprenant l'étape consistant à transférer la matrice ordonnée de macromolécules sur un support.
